# EUROPEAN PATENT APPLICATION

(11) **EP 4 631 439 A1**
(43) Date of publication of application: **15.10.2025**
(21) Application number: 23888985.1
(22) Date of filing: 25.10.2023
(51) Int. Cl.: A61B 10/00, A61B 5/00, A61B 5/349, A61B 5/16, G16H 50/20, G16H 10/20, G06N 20/20

(54) **METHOD AND DEVICE FOR PROVIDING MENSTRUATION-RELATED INFORMATION**

(30) Priority: 08.11.2022 KR 20220147623
(71) Applicant: University Industry Foundation, Yonsei University Wonju Campus, Gangwon-do 26493 (KR); Mezoo Co., Ltd., Gangwon-do 26354 (KR)
(72) Inventor: LEE, Sanhui, Wonju-si, Gangwon-do 26421 (KR); AHN, Junghwan, Wonju-si, Gangwon-do 26458 (KR); PARK, Sunhwa, Wonju-si, Gangwon-do 26417 (KR); LIM, Seungju, Wonju-si, Gangwon-do 26354 (KR); YOO, Heesung, Wonju-si, Gangwon-do 26394 (KR); PARK, Junghwan, Wonju-si, Gangwon-do 26494 (KR); CHO, Sungpil, Wonju-si Gangwon-do 26494 (KR)
(74) Representative: van Trier, Norbertus Henricus Gerardus
(86) International application number: PCT/KR2023/016651
(87) International publication number: WO 2024/101723

(57) **Abstract**

According to some exemplary embodiments of the present disclosure, disclosed is a method for providing menstrual related information, which is performed by a computing device. The method may include: obtaining biometric information including a plurality of predetermined electrocardiogram variable information, the plurality of electrocardiogram variable information including time series data; obtaining user question and answer information corresponding to the plurality of electrocardiogram variable information; and generating, by an analysis model, menstrual cycle prediction information based on the biometric information and the user question and answer information.

## Description

### CROSS-REFERENCE TO RELATED APPLICATIONS

This application claims priority to and the benefit of Korean Patent Application No. 10-2022-0147623 filed in the Korean Intellectual Property Office on November 08, 2022, the entire contents of which are incorporated herein by reference.

### TECHNICAL FIELD

The present disclosure relates to a method and a device for providing information using a computer, and more particularly, to a method and a device for providing menstrual related information using biometric information including a plurality of electrocardiogram (ECG) variable information and user question and answer information.

### BACKGROUND ART

A commonly known menstrual cycle prediction method predicts an ovulation day (menstrual period) only with a body temperature of a woman, and the accuracy of measurement is not high. Therefore, there is a problem in that accurate menstrual related information such as fertile phase information and menstrual disorder prevention and alleviation information cannot be provided due to inaccurate prediction results.

### SUMMARY OF THE INVENTION

The present disclosure is contrived in response to the foregoing background art, and has been made in an effort to provide a method and a device for providing menstrual related information by using biometric information including a plurality of electrocardiogram variable information and user question and answer information.

An exemplary embodiment of the present disclosure provides a method for providing menstrual related information, which is performed by a computing device. The method may include: obtaining biometric information including a plurality of predetermined electrocardiogram variable information, the plurality of electrocardiogram variable information including time series data; obtaining user question and answer information corresponding to the plurality of electrocardiogram variable information; and generating, by an analysis model, menstrual cycle prediction information based on the biometric information and the user question and answer information.

Alternatively, the plurality of electrocardiogram variable information may include at least one of RR Intervals (RRI) information, heart rate (HR) information, respiration rate (RR) information, ST level information, standard deviation of all NN intervals (SDNN) information, square root of the mean of the sum of the squares of differences between adjacent NN intervals (RMSSD) information, number of NN intervals differing by more than 50 ms (NN50) information, ratio of NN50 (pNN50) information, and standard deviation of differences between neighboring NN intervals (SDSD) information.

Alternatively, the user question and answer information may include at least one of sleep disorder question and answer information, depression question and answer information, anxiety question and answer information, and stress question and answer information.

Alternatively, the analysis model may include an ensemble learning model using a plurality of prediction variable combinations including at least some of the plurality of electrocardiogram variable information and the user question and answer information.

Alternatively, the analysis model may be trained to generate information predicting a transition from a follicular phase to a luteal phase when identifying a decreasing trend of the RRI information based on the RRI information.

Alternatively, the analysis model may be trained to generate information predicting a transition from a menstrual phase to the follicular phase when identifying a retention decreasing trend of the HR information, trained to generate information predicting a transition from the follicular phase to a fertile phase when identifying an increasing trend of HR information, and trained to generate information predicting a transition to the luteal phase when identifying a highest level trend of the HR information.

Alternatively, the analysis model may be trained to generate information predicting a transition from the menstrual phase to the follicular phase when identifying a decreasing trend of the RR information, trained to generate information predicting a transition to the fertile phase when identifying an additional decreasing trend of the RR information, and trained to generate information predicting a transition from the luteal phase when identifying an increasing trend of the RR information.

Alternatively, the analysis model may be trained to generate information predicting a transition to the follicular phase when identifying a highest level trend of the ST level information, and trained to generate information predicting a transition to the luteal phase when identifying a lowest level trend of the ST level information.

Alternatively, the analysis model may be trained to generate information predicting a transition from the menstrual phase to the follicular phase when identifying a retention trend of the SDNN information, trained to generate information predicting a transition from the follicular phase to the fertile phase when identifying a decreasing trend of the SDNN information, and trained to generate information predicting a transition to the luteal phase when identifying a maximum decreasing trend of the SDNN information.

Alternatively, the analysis model may be trained to generate information predicting a transition to the luteal phase when identifying a maximum decreasing trend of the RMSSD information.

Alternatively, the analysis model may be trained to generate information predicting a transition from the follicular phase to the luteal phase when identifying a decreasing trend of the NN50 information.

Alternatively, the analysis model may be trained to generate information predicting a transition from the follicular phase to the luteal phase when identifying a decreasing trend of the pNN50 information.

Alternatively, the analysis model may be trained to generate information predicting a transition from the menstrual phase to the follicular phase when identifying a retention trend of the SDSD information.

Alternatively, the user question and answer information may include sleep disorder question and answer information, and the analysis model may be trained to generate information predicting a transition to the luteal phase when identifying an increasing trend of the sleep disorder question and answer information.

Alternatively, the user question and answer information may include depression question and answer information, and the analysis model may be trained to generate information predicting a transition to the luteal phase when identifying an increasing trend of the depression question and answer information.

Alternatively, the user question and answer information may include anxiety question and answer information, and the analysis model may be trained to generate information predicting a transition to the luteal phase when identifying an increasing trend of the anxiety question and answer information.

Alternatively, the user question and answer information may include stress question and answer information, and the analysis model may be trained to generate information predicting an ovulation delay when identifying an increasing trend of the stress question and answer information.

Alternatively, the obtaining of the user question and answer information corresponding to the plurality of electrocardiogram variable information may include providing a user interface, capable of inputting the user question and answer information, corresponding to time points of obtaining the plurality of electrocardiogram variable information.

Alternatively, the obtaining of the user question and answer information corresponding to the plurality of electrocardiogram variable information may include providing a user interface indicating whether to input the user question and answer information corresponding to the time points of obtaining the plurality of electrocardiogram variable information.

Alternatively, the plurality of electrocardiogram variable information may include RRI information, HR information, RR information, ST level information, SDNN information, RMSSD information, NN50 information, pNN50 information, and SDSD information.

Alternatively, the user question and answer information may include sleep disorder question and answer information, depression question and answer information, anxiety question and answer information, and stress question and answer information.

Alternatively, the analysis model may use a random forest algorithm.

Another exemplary embodiment of the present disclosure provides a computer program stored in a computer readable medium. The computer program may include instructions allowing one or processors to perform a method for providing menstrual related information, and the method may include: obtaining biometric information including a plurality of predetermined electrocardiogram variable information, the plurality of electrocardiogram variable information including time series data; obtaining user question and answer information corresponding to the plurality of electrocardiogram variable information; and generating, by an analysis model, menstrual cycle prediction information based on the biometric information and the user question and answer information.

Yet another exemplary embodiment of the present disclosure provides a computing device performing a method for providing menstrual related information. The computing device may include: a memory including computer-executable components; and a processor executing following computer-executable components stored in the memory, and the processor may be configured to obtain biometric information including a plurality of predetermined electrocardiogram variable information, the plurality of electrocardiogram variable information including time series data, obtain user question and answer information corresponding to the plurality of electrocardiogram variable information, and generate, by an analysis model, menstrual cycle prediction information based on the biometric information and the plurality of user question and answer information.

According to exemplary embodiments of the present disclosure, it is possible to provide a method and a device for providing menstrual related information by using biometric information including a plurality of electrocardiogram variable information and user question and answer information.

### BRIEF DESCRIPTION OF THE DRAWINGS

FIG. 1 is a diagram illustrating a system for providing menstrual related information according to some exemplary embodiments of the present disclosure.
FIG. 2 is a block diagram of a computing device for performing a method for providing menstrual related information according to some exemplary embodiments of the present disclosure.
FIG. 3 is a schematic diagram for describing the method for providing menstrual related information according to some exemplary embodiments of the present disclosure.
FIG. 4 is a diagram for describing electrocardiogram variable information according to some exemplary embodiments of the present disclosure.
FIG. 5 is a diagram for describing user question and answer information according to some exemplary embodiments of the present disclosure.
FIG. 6 illustrates an exemplary user application for obtaining the user question and answer information according to some exemplary embodiments of the present disclosure.
FIG. 7 is a diagram for describing a prediction performance of an analysis model according to some exemplary embodiments of the present disclosure.
FIG. 8 is another diagram for describing the prediction performance of the analysis model according to some exemplary embodiments of the present disclosure.
FIG. 9 is a flowchart of the method for providing menstrual related information according to some exemplary embodiments of the present disclosure.
FIG. 10 is a schematic diagram illustrating a network function according to some exemplary embodiments of the present disclosure.
FIG. 11 is a block diagram of a computing device according to some exemplary embodiments of the present disclosure.
Fig. 12 is a graph analyzing the change pattern identified in the RRI information to give information about the menstrual cycle.

### DETAILED DESCRIPTION

Various exemplary embodiments will now be described with reference to drawings. In this specification, various descriptions are presented to provide appreciation of the present disclosure. However, it is apparent that the embodiments can be executed without the specific description.

"Component", "module", "system", and the like which are terms used in the specification refer to a computer-related entity, hardware, firmware, software, and a combination of the software and the hardware, or execution of the software. For example, the component may be a executed on a processor, the processor, an object, an execution thread, a program, and/or a computer, but is not limited thereto. For example, both an application executed in a computing device and the computing device may be the components. One or more components may reside within the processor and/or an execution thread. One component may be localized in one computer. One component may be distributed between two or more computers. Further, the components may be executed by various computer-readable media having various data structures, which are stored therein. The components may perform communication through local and/or remote processing according to a signal (for example, data transmitted from another system through a network such as the Internet through data and/or a signal from one component that interacts with other components in a local system and a distribution system) having one or more data packets, for example.

In addition, the term "or" is intended to mean not exclusive "or" but implicit "or". That is, when not separately specified or not clear in terms of a context, a sentence "X uses A or B" is intended to mean one of the natural inclusive replacements. That is, the sentence "X uses A or B" may be applied to any of the case where X uses A, the case where X uses B, or the case where X uses both A and B. Further, it should be understood that the term "and/or" used in this specification designates and includes all available combinations of one or more items among enumerated related items.

Further, it should be appreciated that the term "comprise" and/or "comprising" means presence of corresponding features and/or components. However, it should be appreciated that the term "comprises" and/or "comprising" means that presence or addition of one or more other features, components, and/or a group thereof is not excluded. Further, when not separately specified or it is not clear in terms of the context that a singular form is indicated, it should be construed that the singular form generally means "one or more" in this specification and the claims.

In addition, the term "at least one of A or B" should be interpreted to mean "a case including only A", "a case including only B", and "a case in which A and B are combined".

Those skilled in the art need to recognize that various illustrative logical blocks, configurations, modules, circuits, means, logic, and algorithm steps described in connection with the exemplary embodiments disclosed herein may be additionally implemented as electronic hardware, computer software, or combinations of both sides. To clearly illustrate the interchangeability of hardware and software, various illustrative components, blocks, configurations, means, logic, modules, circuits, and steps have been described above generally in terms of their functionalities. Whether the functionalities are implemented as the hardware or software depends on a specific application and design restrictions given to an entire system. Skilled artisans may implement the described functionalities in various ways for each particular application. However, such implementation decisions should not be interpreted as causing a departure from the scope of the present disclosure.

The description of the presented exemplary embodiments is provided so that those skilled in the art of the present disclosure use or implement the present disclosure. Various modifications to the exemplary embodiments will be apparent to those skilled in the art. Generic principles defined herein may be applied to other exemplary embodiments without departing from the scope of the present disclosure. Therefore, the present disclosure is not limited to the exemplary embodiments presented herein. The present disclosure should be analyzed within the widest range which is coherent with the principles and new features presented herein.

The present disclosure may provide a method for providing menstrual related information with high accuracy by using electrocardiogram variable information and user question and answer information, without being limited to a body temperature, which has been mainly used as a variable for predicting a menstrual cycle. Specifically, the present disclosure may provide menstrual related information by identifying a time-series change pattern on electrocardiogram variable information, which is time-series data collected continuously for a certain period of time. In addition, the present disclosure may provide the menstrual-related information with higher accuracy by using the user question and answer information obtained in the form of a question and answer from a user together with the electrocardiogram variable information.

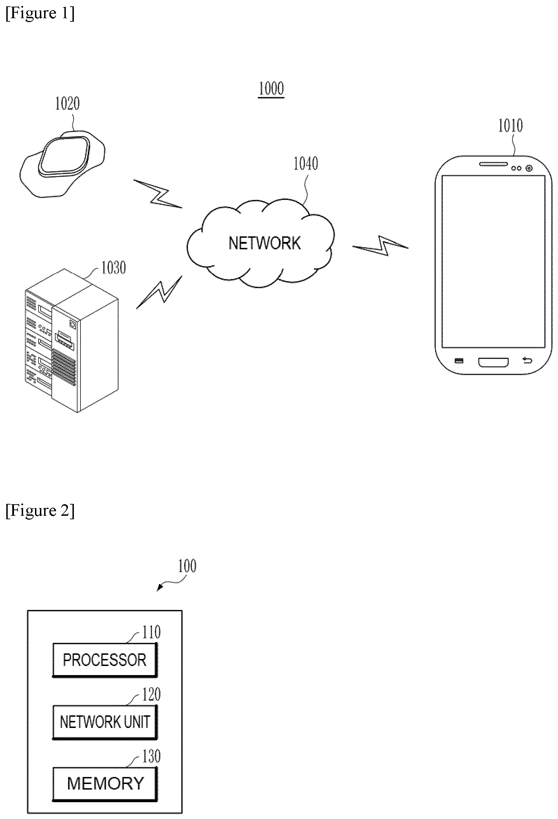

FIG. 1 is a diagram illustrating a system 1000 for providing menstrual related information according to some exemplary embodiments of the present disclosure.

Referring to FIG. 1, a menstrual information providing system 1000 according to some exemplary embodiments of the present disclosure may include a user terminal 1010, a biometric information sensing device 1020, and a server 1030. A configuration of the menstrual information providing system 1000 illustrated in FIG. 1 is only an example illustrated through simplification. In some exemplary embodiments of the present disclosure, the menstrual information providing system 1000 may include other additional components, and only some of the disclosed components may also constitute the menstrual information providing system 1000.

Communication between various entities included in the menstrual related information providing system 1000 may be performed via a wired/wireless network 1040. Here, the wired/wireless network 1040 may refer to a connection structure in which information may be exchanged between respective nodes such as a plurality of terminals and servers. Some examples of such networks 1040 may include a Local Area Network (LAN), a Wide Area Network (WAN), a World Wide Web (WWW), a wired and wireless data network, a telephone network, a wired and wireless television network, and the like. Some examples of the wireless communication network include 3G, 4G, 5G, 3rd Generation Partnership Project (3GPP), 5th Generation Partnership Project (5GPP), Long Term Evolution (LTE), World Interoperability for Microwave Access (WIMAX), Wi-Fi, Internet, Local Area Network (LAN), Wireless Local Area Network (LAN), Wide Area Network (WAN), Personal Area Network (PAN), radio frequency (RF), Bluetooth network, Near-Field Communication (NFC) network, satellite broadcasting network, analog broadcasting network, Digital Multimedia Broadcasting (DMB) network, and the like, etc., but are not limited thereto.

The user terminal 1010 may be implemented as a computer capable of connecting to a remote server or terminal through a network. Here, the user terminal 1010 may include at least one of a smartphone, a tablet personal computer, a mobile phone, a video phone, an e-book reader, a desktop personal computer, a laptop personal computer, a netbook computer, a workstation, a server, a personal digital assistant (PDA), a portable multimedia player (PMP), or a wearable device (e.g., smart glasses, a head-mounted-device (HMD), an electronic garment, an electronic bracelet, an electronic necklace, an electronic accessory, a smart mirror, or a smart watch). However, the present disclosure is not limited thereto, and the user terminal 1010 may be implemented by various computers.

According to some exemplary embodiments of the present disclosure, the user terminal 1010 may implement a method for providing menstrual related information according to the present disclosure. For example, the user terminal 1010 may provide a user application for providing the menstrual related information. The user terminal 1010 may obtain menstrual related user information for providing the menstruation related information through the user application. In some examples, the user terminal 1010 may obtain biometric information by operating the biometric information sensing device 1020 through the user application. In some examples, the user terminal 1010 may obtain biometric information stored on the server 1030. In some examples, the user terminal 1010 may obtain user question and answer information input through the user interface of the user application.

In some examples, the user terminal 1010 may generate the menstrual related user information using an analysis model implemented on the user terminal 1010. Alternatively, the user terminal 1010 may transmit the menstrual related user information to the server 1030 to process the obtained data. In this case, the user terminal 1010 may receive the menstrual related information generated by processing the menstruation related user information using the analysis model provided in the server 1030. The user terminal 1010 may provide the received menstrual related information to the user through the user application.

The menstrual related information may include various types of information related to menstruation. For example, the menstrual related information may include a predicted menstrual cycle (e.g., days of ovulation, menstrual days, periods of menstruation, gestational days, premenstrual syndrome, menopause, etc.), a recommended diet, guide information for menstrual management (for example, predicted menstruation disorders and music therapy and exercise therapy for alleviating menstrual disorders, etc.), or recommended menstrual product and service information, etc. The type of menstrual related information is not limited to the above-described example, and may include various types of information related to menstruation.

According to some exemplary embodiments of the present disclosure, the server 1030 may implement a method for providing the menstrual related information according to the present disclosure. For example, the server 1030 may train the analysis model using menstrual related training data. In some examples, menstrual cycle related training data may include various menstrual related user information including biometric information and user question and answer information. The server 1030 may generate the menstrual related information by processing the menstrual related user information using the trained analysis model. As another example, the server 1030 may provide a user application including the trained analysis model to the user terminal 1010. In this case, as described above, the user terminal 1010 may process the menstrual related user information by using the analysis model.

The biometric information sensing device 1020 may include various devices capable of measuring biometric information. For example, the biometric information sensing device 1020 may be a device capable of measuring a user's body temperature, blood pressure, pulse, skin conductivity, electrocardiogram, oxygen saturation, respiratory rate, and the like. The biometric information sensing device 1020 may transmit the measured biometric information to the user terminal 1010 or the server 1030. The biometric information sensing device 1020 may include a portable device or a stationary device. In some examples, the biometric information sensing device 1020 may include a wearable device (e.g., a Holter electrocardiogram in the form of a patch) capable of measuring an electrocardiogram. In some examples, the biometric information sensing device 1020 may be modularly provided on the user terminal 1010.

Hereinafter, a computer device implementing a method for providing the menstrual related information according to the present disclosure, which is a user terminal 1010 or a server 1030, will be described according to some exemplary embodiments of the present disclosure.

FIG. 2 is a block diagram of a computing device for performing a method for providing menstrual related information according to some exemplary embodiments of the present disclosure.

As illustrated in FIG. 2, the computing device 100 may include a processor 110, a memory 130, and a network unit 120. A configuration of the computing device 100 illustrated in FIG. 2 is only a simplified example. In some exemplary embodiments of the present disclosure, the computing device 100 may include other components for performing a computing configuration of the computing device 100 and only some of the disclosed components may constitute the computing device 100. Computing device

The processor 110 may be constituted by one or more cores and may include processors for data analysis and processing, and deep learning, which include a central processing unit (CPU), a general purpose graphics processing unit (GPGPU), a tensor processing unit (TPU), and the like of the computing device. The processor 110 may read a computer program stored in the memory 130 to perform data conversion, operation, generation, etc., for providing menstrual related information according to some exemplary embodiments of the present disclosure. For example, the processor 110 may perform steps for performing a method for providing menstrual related information described below. Further, according to some exemplary embodiments of the present disclosure, the processor 110 may perform an operation for training a neural network by using training data in order to perform the method for providing menstrual related information. For example, the processor 110 may generate/train the analysis model using training data. The processor 110 may perform calculations for training the neural network, which include processing of input data for learning in deep learning (DL), extracting a feature in the input data, calculating an error, updating a weight of the neural network using backpropagation, and the like. At least one of the CPU, the GPGPU, and the TPU of the processor 110 may process an operation for the method for providing menstrual related information. For example, the CPU and the GPGPU may jointly process the operation for the method for providing menstrual related information. Further, in some exemplary embodiments of the present disclosure, processors of a plurality of computing devices may be used together to process data conversion, operation, and generation for the method for providing menstrual related information, the learning of the network function and the data classification using the network function. Further, the computer program executed in the computing device according to some exemplary embodiments of the present disclosure may be a CPU, GPGPU, or TPU executable program.

According to some exemplary embodiments of the present disclosure, the memory 130 may store any type of information generated or determined by the processor 110 or any type of information received by the network unit 120. For example, the memory 130 may store data generated in the process of performing the method for providing menstrual related information by the processor 110. Further, the memory 130 may store data received from the outside in the process of performing the method for providing menstrual related information by the processor 110. However, without being not limited thereto, and the memory 130 may store various information for performing the method for providing menstrual related information according to some exemplary embodiments of the present disclosure.

According to some embodiments of the present disclosure, the memory 130 may include at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The above description of the memory is just an example and the present disclosure is not limited thereto.

The network unit 120 according to some exemplary embodiments of the present disclosure may use an arbitrary type of known wired/wireless communication system.

The network unit 120 may transmit and receive information processed by the processor 110, a user interface, and the like through communication with other terminals. For example, the network unit 120 may provide the user interface generated by the processor 110 to a client (e.g., a user terminal). In addition, the network unit 120 may receive an external input of a user applied to a client and transfer the external input to the processor 110. In this case, the processor 110 may process operations such as outputting, correcting, changing, adding, and the like of information provided through the user interface based on the external input of the user received from the network unit 120.

Specifically, for example, the network unit 120 may transmit and receive various information for performing the method for providing menstrual related information according to some exemplary embodiments of the present disclosure. For example, the network unit 120 may transmit and receive biometric information and user question and answer information stored on the database. Additionally, the network unit 120 may externally transmit some data generated in the process of performing the method for providing menstrual related information described below to be stored in the database.

Meanwhile, according to some exemplary embodiments of the present disclosure, the computing device 100 may include a server 1030 as a computing system that transmits and receives information through communication with the client. In this case, the client may be any type of terminal which may access the server. For example, the computing device 100 which is the server may receive a query from a user terminal and generate a single information processing result corresponding to the query. In this case, the computing device 100 which is the server may provide, to the user terminal, a user interface including the processing result. In this case, the user terminal may output the user interface received from the computing device 100 as the server, and receive or process information through interaction with the user.

In an additional exemplary embodiment, the computing device 100 may also include any type of user terminal 1010 that receives data resources generated by an arbitrary server and performs additional information processing.

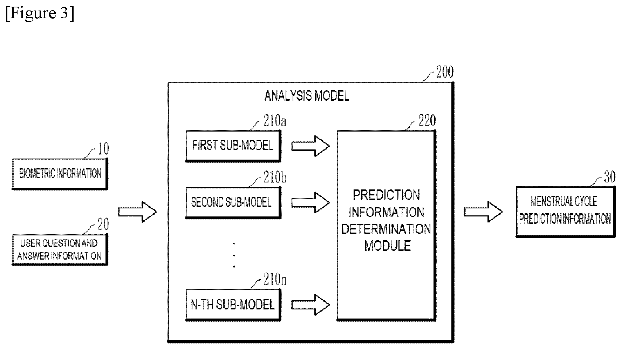

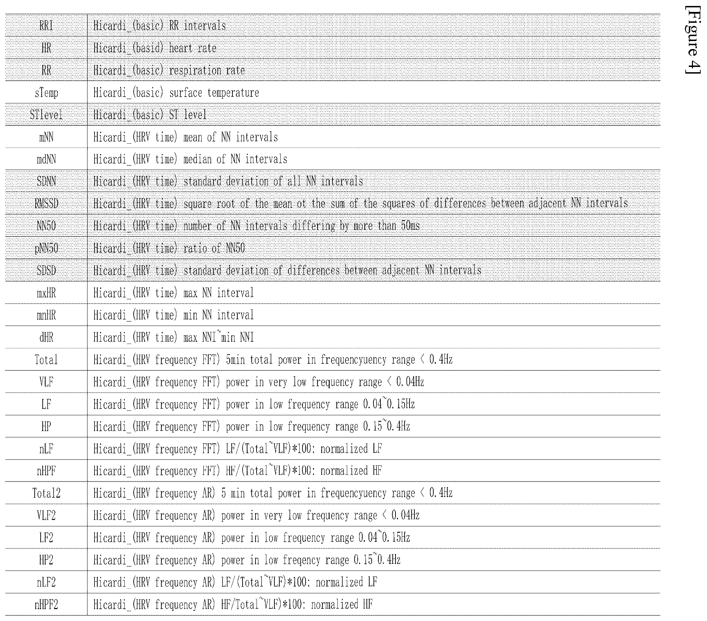

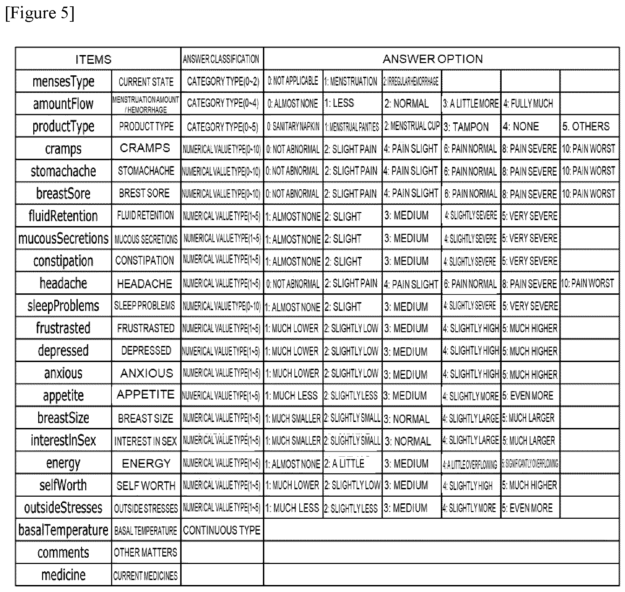

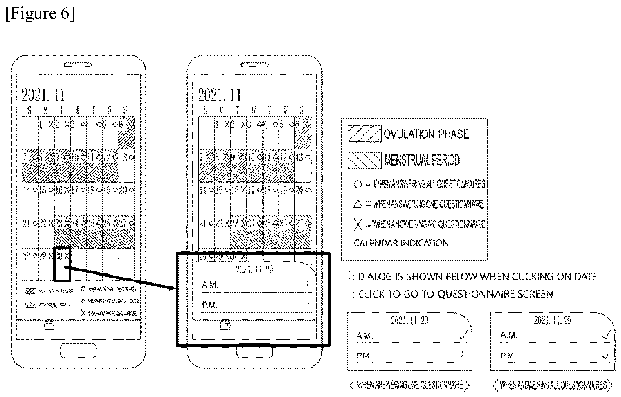

FIG. 3 is a schematic diagram for describing the method for providing menstrual related information according to some exemplary embodiments of the present disclosure. FIG. 4 is a diagram for describing electrocardiogram variable information according to some embodiments of the present disclosure. FIG. 5 is a diagram for describing user question and answer information according to some exemplary embodiments of the present disclosure. FIG. 6 illustrates an exemplary user application for obtaining the user question and answer information according to some exemplary embodiments of the present disclosure.

Referring to FIGS. 3 to 6, an exemplary embodiment according to some exemplary embodiments of the present disclosure is specifically described.

The processor 110 may obtain biometric information including a plurality of predetermined electrocardiogram variable information. Here, the plurality of electrocardiogram variable information may include time series data.

Specifically, the processor 110 may obtain biometric information 10 that may be used to predict a menstrual cycle. For example, the biometric information may include age, height, weight, body temperature, blood pressure, respiratory rate, menstrual day, menstruation period, pulse, skin conductivity, electrocardiogram, oxygen saturation, and the like. However, the biometric information 10 is not limited to the above-described example, and may include various pieces of information related to the user.

In some examples, the biometric information 10 may include information (e.g., body temperature, blood pressure, respiratory rate, pulse, electrocardiogram, etc.) generated by one or more biometric information sensing devices 1020. In addition, the biometric information 10 may include information input by a user (e.g., age, height, weight, etc.).

In some examples, the biometric information 10 may include a plurality of electrocardiogram variable information. For example, the electrocardiogram variable information may be time-series data of an electrocardiogram (EKG or ECG) measured by an electrocardiogram device such as a Holter electrocardiogram. The electrocardiogram variable information may be time-series data obtained by a method for continuously measuring an electrocardiogram rather than a transversal measurement value.

According to some exemplary embodiments of the present disclosure, the plurality of electrocardiogram variable information may include at least one of RR Intervals (RRI) information, heart rate (HR) information, respiration rate (RR) information, ST level information, standard deviation of all NN intervals (SDNN) information, square root of the mean of the sum of the squares of differences between adjacent NN intervals (RMSSD) information, number of NN intervals differing by more than 50 ms (NN50) information, ratio of NN50 (pNN50) information, and standard deviation of differences between neighboring NN intervals (SDSD) information.

Referring to FIG. 4, exemplary electrocardiogram variable information obtained by the halter electrocardiogram is illustrated. Among these, prediction factors processed by the analysis model for the menstrual cycle prediction information may include RRI information, HR information, RR information, ST level information, SDNN information, RMSSD information, NN50 information, pNN50 information, and SDSD information that are analyzed to have a high correlation with the menstruation cycle prediction. The electrocardiogram variable information is not limited to the above-described example, and may include various information related to the electrocardiogram.

The plurality of electrocardiogram variable information may be information obtained according to a predetermined cycle. For example, the plurality of electrocardiogram variable information may be one of the information measured every morning and afternoon. In this case, the analysis model may increase prediction accuracy by generating menstrual cycle prediction information every predetermined cycle. However, without being limited thereto, the plurality of electrocardiogram variable information may be information obtained at various cycles..

The processor 110 may obtain user question and answer information corresponding to the plurality of electrocardiogram variable information.

Specifically, the processor 110 may obtain user question and answer information processed by the analysis model together with the plurality of electrocardiogram variable information. The user question and answer information may include information related to menstrual symptoms. For example, the user question and answer information may be information input by the user on a physical change, a mental change, a menstrual pain/breast pain/head pain change.

According to some exemplary embodiments of the present disclosure, the user question and answer information may include at least one of sleep disorder question and answer information, depression question and answer information and anxiety question and answer information.

FIG. 5 illustrates exemplary user question and answer information. As illustrated in FIG. 5, the user question and answer information may be information input categorically or numerically for various items for a physical change, a mental change, and a menstrual pain/breast pain/head pain change. Among them, the user question and answer information processed by the analysis model for the menstrual cycle prediction information may include sleep disorder question and answer information, depression question and answer information and anxiety question and answer information analyzed to have a high correlation with the menstruation cycle prediction. However, without being limited thereto, the user question and answer information may include various information on menstrual symptoms obtained from the user.

According to some exemplary embodiments of the present disclosure, in a case of obtaining the user question and answer information corresponding to the plurality of electrocardiogram variable information, the processor 110 may provide a user interface capable of inputting the plurality of user question and answer Information in response to time points of obtaining the plurality of piece of electrocardiogram variable information.

As described above, the processor 110 may provide a user interface capable of inputting the user question and answer information through the user application. In general, in order to reduce data throughput, the analysis model generates a prediction result by using information obtained at some time points instead of using information obtained for 24 hours a day, so that the electrocardiogram variable information and the user question and answer information need to be processed between information obtained for the same period. For example, the plurality of electrocardiogram variable information measured this morning may be matched with user question and answer information input for the menstrual symptoms of the user this morning. In addition, the analysis model may generate the menstrual cycle prediction information by processing the matched plurality of electrocardiogram variable information and the user question and answer information together. Therefore, the user question and answer information may be obtained according to a cycle at which the plurality of electrocardiogram variable information is obtained, so as to be processed together with the plurality of electrocardiogram variable information. For example, when the plurality of electrocardiogram variable information are obtained in the morning and afternoon of each day, the processor 110 may provide a user interface capable of inputting the user question and answer information in the morning and the afternoon of each day.

In this regard, the processor 110 may provide a user interface through which the user may input the user question and answer information according to a predetermined cycle at which the plurality of electrocardiogram variable information is obtained. Referring to FIG. 6, an exemplary user interface is illustrated that is capable of inputting the user question and answer information in the user application provided via the user terminal 1010. In some examples, when the cycles of obtaining the plurality of electrocardiogram variable information is two times each in the morning and the afternoon, the user interface may indicate whether the user question and answer information is input in the morning and in the afternoon for each date on a calendar. When receiving an input for selecting a date on which no user question and answer information is input, the user interface may provide an input window for inputting the user question and answer Information on the date. Referring to FIG. 6, when the user interface identifies a user command that selects a specific date on the calendar, the user interface may indicate whether the user question and answer information is completed through a check indication for the morning and afternoon on the date.

In some examples, the user interface may provide an indication that may identify how many pieces of information is input among a plurality of user question and answer information. For example, referring to FIG. 6, the user interface may display an icon indicating the number of completed inputs from among the plurality of user question and answer information for each date on the calendar. However, without being limited thereto, the user interface may be provided in various forms.

According to some exemplary embodiments of the present disclosure, the processor 110 may generate, by the analysis model, menstrual cycle prediction information 30 based on biometric information 10 and user question and answer information 20.

The analysis model may generate the menstrual cycle prediction information by processing biometric information including a plurality of electrocardiogram variable information and a plurality of user question and answer information. The menstrual cycle may be formed as a cycle in which a luteal phase which is a period before the start of menstruation, a menstrual phase which is a period during which menstruation is made, and a follicular phase which is a period after the end of menstrual period are repeated. A fertile phase may refer to a pre- and post-period of reproductive 'ovulation' around 14 days after menstruation. The analysis model may generate THE menstrual cycle prediction information 30 that may identify the luteal phase, the menstrual phase, the follicular phase, and the fertility phase by processing input data including the biometric information and the user question and answer information.

The processor 110 may generate menstrual related information by using the generated menstrual cycle prediction information 30. For example, the processor 110 may provide a predicted menstrual cycle (e.g., days of ovulation, menstrual days, periods of menstruation, gestational days, premenstrual syndrome, menopause, etc.), a recommended diet, guide information for menstrual management (for example, predicted menstruation disorders and music therapy and exercise therapy for alleviating menstrual disorders, etc.), or recommended menstrual product and service information, etc., at an appropriate time by using the menstrual cycle prediction information 30. However, without being limited thereto, the processor 110 may provide various pieces of menstrual related information by using the menstrual cycle prediction information 30.

According to some exemplary embodiments of the present disclosure, the analysis model 200 may include an ensemble learning model using a plurality of prediction variable combinations including at least some of a plurality of electrocardiogram variable information and user question and answer information.

Specifically, since the biometric information 10 including the plurality of electrocardiogram variable information and the user question and answer information 20 are the plurality of prediction variables as described above, the analysis model needs to be implemented with an appropriate algorithm capable of processing the plurality of prediction variables as inputs. For example, the analysis model 200 may be an ensemble learning model capable of deriving a more accurate prediction by integrally processing prediction results of multiple sub-models 210 by a prediction information determination module 220. In some examples, the ensemble learning model may be a model using a voting, bagging, or boosting algorithm using a plurality of sub-models. Hereinafter, an exemplary analysis model according to some exemplary embodiments of the present disclosure is described with respect to a model implemented using a random forest algorithm, which is a type of bagging algorithm, but the present disclosure is not limited thereto. In addition, hereinafter, an exemplary embodiment is described in which the prediction variable combinations of the analysis model includes only electrocardiogram variable information among the biometric information 10, but the prediction variable combinations of the analysis model may further include various biometric information 10 other than the electrocardiogram variable information such as respiratory rate, body temperature, etc.

The random forest algorithm may be an ensemble technique that applies training data to multiple decision-making trees to increase training performance. For example, the analysis model 200 may include n sub-models 210a to 210n trained by n training data generated through reconstruction extraction from original training data. In this case, each sub-model may be learned using a prediction variable combination that combines only some of the plurality of prediction variables. For example, a first sub-model 210a may be a model using a prediction variable combination including RRI information, HR information, and RR information among the plurality of electrocardiogram variable information. A second sub-model 210b may be a model using a prediction variable combination including the RRI information and ST level information among the plurality of electrocardiogram variable information, and sleep disorder question and answer information among the user question and answer information. A third sub-model 210c may be a model using a prediction variable combination including NN50 information among the plurality of electrocardiogram variable information, and the sleep disorder question and answer information and anxiety question and answer information among the plurality of electrocardiogram variable information. Here, the prediction variable combination used by each sub-model may be a randomly determined combination or a predetermined combination.

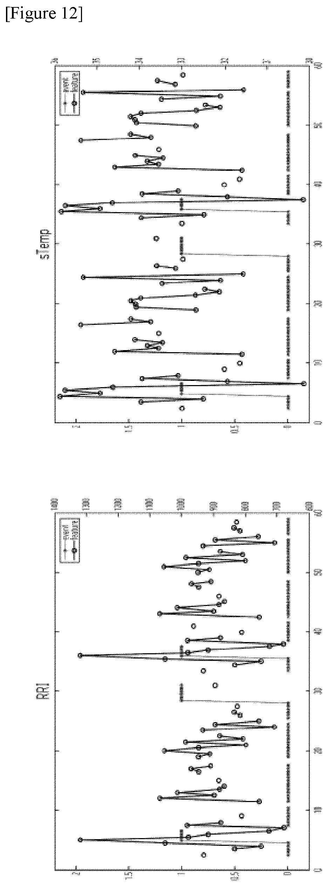

Training data related to the electrocardiogram variable information among the training data for training the analysis model may be generated by assigning a label regarding the menstrual cycle to a change pattern identified in the electrocardiogram variables information, which is time-series data. Fig. 12 is a graph analyzing the change pattern identified in the RRI information to give information about the menstrual cycle.

In some examples, the training data may include a label generated by analyzing a correlation between the change pattern identified in each of the plurality of electrocardiogram variable information and the menstrual cycle. Hereinafter, an exemplary analysis model 200 generated using training data generated according to an analysis result on the correlation between the plurality of electrocardiogram variable information and the menstrual cycle is described according to some exemplary embodiments of the present disclosure. Hereinafter, when the analysis model 200 is an ensemble learning model, training of the analysis model 200 may mean training of an individual sub-model 210.

According to some exemplary embodiments of the present disclosure, for the RRI information, the analysis model 200 may be trained to generate information predicting transition from the follicular phase to the luteal phase when identifying a decreasing trend of the RRI information based on the RRI information. For example, the decreasing trend in RRI information may include a decrease of about 0.15 to 0.40 Hz in the luteal phase compared to the follicular phase.

According to some exemplary embodiments of the present disclosure, for the HR information, the analysis model 200 may be trained to generate information predicting a transition from the menstrual phase to the follicular phase when identifying a retention decreasing trend of the HR information. For example, the retention decreasing trend of HR information may include having a similar value or a decrease of about 1.36 bpm in the follicle phase compared to the menstrual phase.

Further, the analysis model 200 may be trained to generate information predicting a transition from the follicular phase to the fertile phase when identifying an increasing trend of HR information. For example, the increasing trend in HR information may include an increase of about 0.61 bpm in the fertile phase compared the follicular phase.

Further, the analysis model 200 may be trained to generate information predicting a transition to the luteal phase when identifying a highest level trend of the HR information. The highest level trend of the HR information may include an increase of about 2.33 bpm in the luteal phase compared to the follicular phase, an increase of about 1.84 bpm in the luteal phase compared to the fertile phase, and an increase of about 2.31 bpm in the luteal phase compared to the menstrual phase.

According to some exemplary embodiments of the present disclosure, for the RR information, the analysis model 200 may be trained to generate information predicting a transition from the menstrual phase to the follicular phase when identifying a decreasing trend of the RR information. For example, the decreasing trend in RR information may include a decrease of about 0.29 breath/min in the follicular phase compared to the menstrual phase.

Further, the analysis model 200 may be trained to generate information predicting a transition to the fertile phase when identifying an additional decreasing trend of the RR information. For example, the additional decreasing trend of the RR information may include a decrease of about 0.46 breath/min in the fertile phase compared to the menstrual phase.

Further, the analysis model 200 may be trained to generate information predicting a transition from the menstrual phase to the luteal phase when identifying an increasing trend of the RR information. The increasing trend of the RR information may include an increase of about 0.18 breath/min in the luteal phase compared to the menstrual phase.

According to some exemplary embodiments of the present disclosure, for the ST level information, the analysis model 200 may be trained to generate information predicting a transition to the follicular phase when identifying a highest level trend of the ST level information. For example, the highest level trend of the ST level information may include a case in which a highest value is shown among the values of the ST level information.

Further, the analysis model 200 may be trained to generate information predicting a transition to the luteal phase when identifying a lowest level trend of the ST level information. For example, the highest level trend of the ST level information may include a case in which a highest value is shown among the values of the ST level information.

According to some exemplary embodiments of the present disclosure, for the SDNN information, the analysis model 200 may be trained to generate information predicting a transition from the menstrual phase to the follicular phase when identifying a retention trend of the SDNN information.

Further, the analysis model 200 may be trained to generate information that predicts a transition from the follicular phase to the fertile phase when identifying a decreasing trend of the SDNN information. For example, the decreasing trend in SDNN information may include a decrease of about 0.04 in the fertile phase compared the follicular phase.

Further, the analysis model 200 may be trained to generate information predicting a transition to the luteal phase when identifying a maximum decreasing trend of the SDNN information. For example, the maximum decreasing trend of the SDNN information may include a case in which a lowest value is shown among the values of the SDNN information.

According to some exemplary embodiments of the present disclosure, for the RMSSD information, the analysis model 200 may be trained to generate information predicting a transition to the luteal phase when identifying a maximum decreasing trend of the RMSSD information. For example, the maximum decreasing trend of the RMSSD information may include a case in which a lowest value is shown among the values of the RMSSD information.

According to some exemplary embodiments of the present disclosure, for the NN50 information, the analysis model 200 may be trained to generate information predicting a transition from the follicular phase to the luteal phase when identifying a decreasing trend of the NN50 information.

According to some exemplary embodiments of the present disclosure, for the pNN50 information, the analysis model 200 may be trained to generate information predicting a transition from the follicular phase to the luteal phase when identifying a decreasing trend of the pNN50 information.

According to some exemplary embodiments of the present disclosure, for the SDSD information, the analysis model 200 may be trained to generate information predicting a transition from the menstrual phase to the follicular phase when identifying a retention trend of the SDSD information.

In some examples, the training data may further include data generated by analyzing a correlation between the change pattern identified in each of the user question and answer information and the menstrual cycle. Hereinafter, an operation of the exemplary analysis model 200 generated using training data generated according to an analysis result on the correlation between the user question and answer information and the menstrual cycle is described according to some exemplary embodiments of the present disclosure. Hereinafter, when the analysis model 200 is an ensemble learning model, training of the analysis model 200 may mean training of the sub-model 210.

According to some exemplary embodiments of the present disclosure, for the sleep disorder question and answer information, the analysis model 200 may be trained to generate information predicting a transition to the luteal phase when identifying an increasing trend of the sleep disorder question and answer information.

According to some exemplary embodiments of the present disclosure, for the depression question and answer information, the analysis model 200 may be trained to generate information predicting a transition to the luteal phase when identifying an increasing trend of the depression question and answer information.

According to some exemplary embodiments of the present disclosure, for the anxiety question and answer information, the analysis model 200 may be trained to generate information predicting a transition to the luteal phase when identifying an increasing trend of the anxiety question and answer information.

According to some exemplary embodiments of the present disclosure, for the stress question and answer information, the analysis model 200 may be trained to generate information predicting the ovulation delay when identifying an increasing trend of the stress question and answer information.

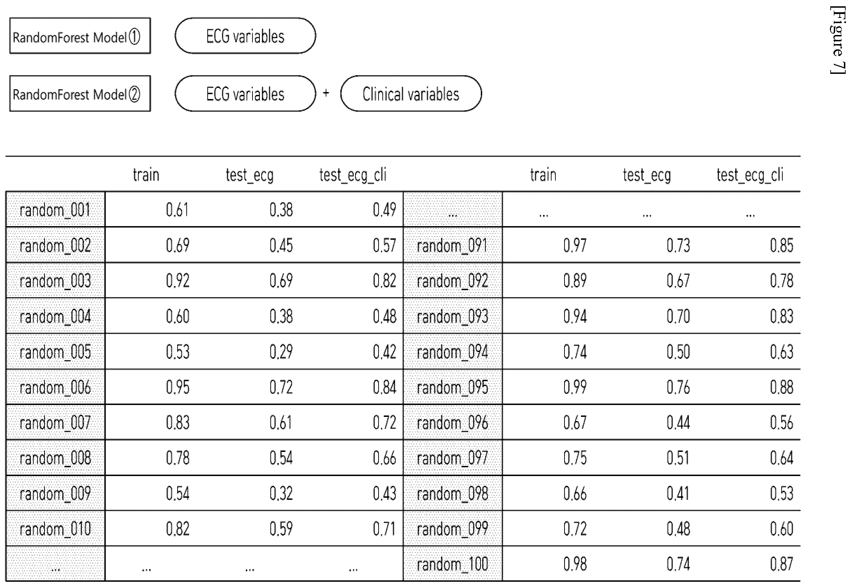

FIG. 7 is a diagram for describing a prediction performance of an analysis model according to some exemplary embodiments of the present disclosure.

FIG. 7 illustrates a prediction performance of an analysis model according to a combination of electrocardiogram variable information and user question and answer information. Referring to FIG. 7, FIG. 7 illustrates that 'random forest model 2' implemented using the electrocardiogram variable information and the user question and answer information together has higher prediction performance than the `random forest model 1' implemented using only the electrocardiogram variables information. Specifically, a prediction performance (test_ecg) of 'random forest model 1' is lower than a prediction performance (test_ecg_cli) of 'random forest model 2'. Therefore, it can be seen that the prediction performance of the analysis model according to the present disclosure is higher by using the electrocardiogram variable information and the user question and answer information together as a prediction factor.

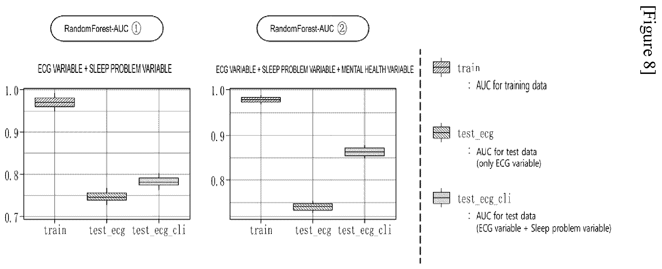

FIG. 8 is another diagram for describing the prediction performance of the analysis model according to some exemplary embodiments of the present disclosure.

FIG. 8 illustrates prediction performance according to a combination of a sleep problem variable (e.g., sleep disorder question and answer information) and a mental health variable (e. g., depression question and answer information, anxiety question and answer information and stress question and answer information), among user question and answer information. Referring to FIG. 8, the prediction performance result (RandomForest-AUC 1) in which only the sleep problem variable in the user question and answer information is used together with the electrocardiogram variable information indicates a lower value than the prediction performance results (RandomForest-AUC 2) in which the sleep problem variable and the mental health variable in the user answer information are used together with the electrocardiogram variable information. Therefore, it can be seen that the prediction performance of the analysis model according to the present disclosure becomes higher by using both the sleep problem variable and the metal health variable as user question and answer information as the prediction factor.

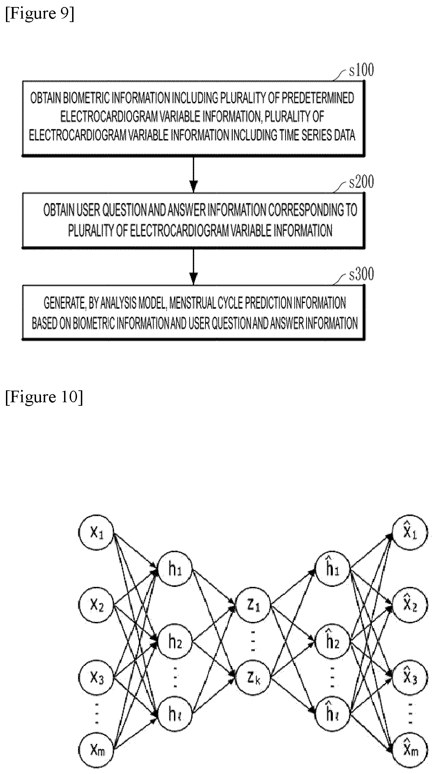

FIG. 9 is a flowchart of the method for providing menstrual related information according to some exemplary embodiments of the present disclosure.

According to some exemplary embodiments of the present disclosure, the method for providing menstrual related information may include a step s100 of obtaining biometric information including a plurality of predetermined electrocardiogram variable information. Here, the plurality of electrocardiogram variable information may include time series data.

According to some exemplary embodiments of the present disclosure, the method for providing menstrual related information may include a step s200 of obtaining user question and answer information corresponding to the plurality of electrocardiogram variable information.

Alternatively, the step s200 of obtaining the user question and answer information corresponding to the plurality of electrocardiogram variable information may include a step of providing a user interface indicating whether to input the user question and answer information corresponding to time points of obtaining the plurality of electrocardiogram variables information.

Alternatively, the step s200 of obtaining the user question and answer information corresponding to the plurality of electrocardiogram variable information may include a step of providing a user interface indicating whether or not to input the user question and question information corresponding to a time point of obtaining the plurality of piece of electrocardiogram variables information.

According to some exemplary embodiments of the present disclosure, the method for providing menstrual related information may include a step s300 of generating, by an analysis model, menstrual cycle prediction information based on biometric information and user question and answer information.

The steps according to the method for providing menstrual related information described above are presented just for description, and some steps may be omitted or separate steps may be added. Further, the above-described steps may be performed according to an arbitrary order.

FIG. 10 is a schematic view illustrating a network function according to an exemplary embodiment of the present disclosure.

Throughout the present specification, a computation model, the neural network, a network function, and the neural network may be used as the same meaning. The neural network may be generally constituted by an aggregate of calculation units which are mutually connected to each other, which may be called nodes. The nodes may also be called neurons. The neural network is configured to include one or more nodes. The nodes (alternatively, neurons) constituting the neural networks may be connected to each other by one or more links.

In the neural network, one or more nodes connected through the link may relatively form the relationship between an input node and an output node. Concepts of the input node and the output node are relative and a predetermined node which has the output node relationship with respect to one node may have the input node relationship in the relationship with another node and vice versa. As described above, the relationship of the input node to the output node may be generated based on the link. One or more output nodes may be connected to one input node through the link and vice versa.

In the relationship of the input node and the output node connected through one link, a value of data of the output node may be determined based on data input in the input node. Here, a link connecting the input node and the output node to each other may have a weight. The weight may be variable and the weight is variable by a user or an algorithm in order for the neural network to perform a desired function. For example, when one or more input nodes are mutually connected to one output node by the respective links, the output node may determine an output node value based on values input in the input nodes connected with the output node and the weights set in the links corresponding to the respective input nodes.

As described above, in the neural network, one or more nodes are connected to each other through one or more links to form a relationship of the input node and output node in the neural network. A characteristic of the neural network may be determined according to the number of nodes, the number of links, correlations between the nodes and the links, and values of the weights granted to the respective links in the neural network. For example, when the same number of nodes and links exist and there are two neural networks in which the weight values of the links are different from each other, it may be recognized that two neural networks are different from each other.

The neural network may be constituted by a set of one or more nodes. A subset of the nodes constituting the neural network may constitute a layer. Some of the nodes constituting the neural network may constitute one layer based on the distances from the initial input node. For example, a set of nodes of which distance from the initial input node is n may constitute n layers. The distance from the initial input node may be defined by the minimum number of links which should be passed through for reaching the corresponding node from the initial input node. However, definition of the layer is predetermined for description and the order of the layer in the neural network may be defined by a method different from the aforementioned method. For example, the layers of the nodes may be defined by the distance from a final output node.

The initial input node may mean one or more nodes in which data is directly input without passing through the links in the relationships with other nodes among the nodes in the neural network. Alternatively, in the neural network, in the relationship between the nodes based on the link, the initial input node may mean nodes which do not have other input nodes connected through the links. Similarly thereto, the final output node may mean one or more nodes which do not have the output node in the relationship with other nodes among the nodes in the neural network. Further, a hidden node may mean nodes constituting the neural network other than the initial input node and the final output node.

In the neural network according to an exemplary embodiment of the present disclosure, the number of nodes of the input layer may be the same as the number of nodes of the output layer, and the neural network may be a neural network of a type in which the number of nodes decreases and then, increases again from the input layer to the hidden layer. Further, in the neural network according to another exemplary embodiment of the present disclosure, the number of nodes of the input layer may be smaller than the number of nodes of the output layer, and the neural network may be a neural network of a type in which the number of nodes decreases from the input layer to the hidden layer. Further, in the neural network according to yet another exemplary embodiment of the present disclosure, the number of nodes of the input layer may be larger than the number of nodes of the output layer, and the neural network may be a neural network of a type in which the number of nodes increases from the input layer to the hidden layer. The neural network according to still yet another exemplary embodiment of the present disclosure may be a neural network of a type in which the neural networks are combined.

A deep neural network (DNN) may refer to a neural network that includes a plurality of hidden layers in 3ddition to the input and output layers. When the deep neural network is used, the latent structures of data may be determined. That is, latent structures of photos, text, video, voice, and music (e.g., what objects are in the photo, what the content and feelings of the text are, what the content and feelings of the voice are) may be determined. The deep neural network may include a convolutional neural network (CNN), a recurrent neural network (RNN), an auto encoder, generative adversarial networks (GAN), a restricted Boltzmann machine (RBM), a deep belief network (DBN), a Q network, a U network, a Siam network, a Generative Adversarial Network (GAN), Transformer and the like. The description of the deep neural network described above is just an example and the present disclosure is not limited thereto.

The neural network may be learned in at least one scheme of supervised learning, unsupervised learning, semi supervised learning, or reinforcement learning. The learning of the neural network may be a process in which the neural network applies knowledge for performing a specific operation to the neural network.

The neural network may be learned in a direction to minimize errors of an output. The learning of the neural network is a process of repeatedly inputting learning data into the neural network and calculating the output of the neural network for the learning data and the error of a target and back-propagating the errors of the neural network from the output layer of the neural network toward the input layer in a direction to reduce the errors to update the weight of each node of the neural network. In the case of the supervised learning, the learning data labeled with a correct answer is used for each learning data (i.e., the labeled learning data) and in the case of the unsupervised learning, the correct answer may not be labeled in each learning data. That is, for example, the learning data in the case of the supervised learning related to the data classification may be data in which category is labeled in each learning data. The labeled learning data is input to the neural network, and the error may be calculated by comparing the output (category) of the neural network with the label of the learning data. As another example, in the case of the unsupervised learning related to the data classification, the learning data as the input is compared with the output of the neural network to calculate the error. The calculated error is back-propagated in a reverse direction (i.e., a direction from the output layer toward the input layer) in the neural network and connection weights of respective nodes of each layer of the neural network may be updated according to the back propagation. A variation amount of the updated connection weight of each node may be determined according to a learning rate. Calculation of the neural network for the input data and the back-propagation of the error may constitute a learning cycle (epoch). The learning rate may be applied differently according to the number of repetition times of the learning cycle of the neural network. For example, in an initial stage of the learning of the neural network, the neural network ensures a certain level of performance quickly by using a high learning rate, thereby increasing efficiency and uses a low learning rate in a latter stage of the learning, thereby increasing accuracy.

To increase the amount of training data for neural networks, various data augmentation methods can be used. For example, data augmentation can be performed through two-dimensional transformations such as rotation, scaling, shearing, reflection, and translation. In addition, data augmentation can be carried out by applying techniques such as noise injection, color adjustment, and brightness variation. In learning of the neural network, the learning data may be generally a subset of actual data (i.e., data to be processed using the learned neural network), and as a result, there may be a learning cycle in which errors for the learning data decrease, but the errors for the actual data increase. Overfitting is a phenomenon in which the errors for the actual data increase due to excessive learning of the learning data. For example, a phenomenon in which the neural network that learns a cat by showing a yellow cat sees a cat other than the yellow cat and does not recognize the corresponding cat as the cat may be a kind of overfitting. The overfitting may act as a cause which increases the error of the machine learning algorithm. Various optimization methods may be used in order to prevent the overfitting. In order to prevent the overfitting, a method such as increasing the learning data, regularization, dropout of omitting a part of the node of the network in the process of learning, utilization of a batch normalization layer, etc., may be applied.

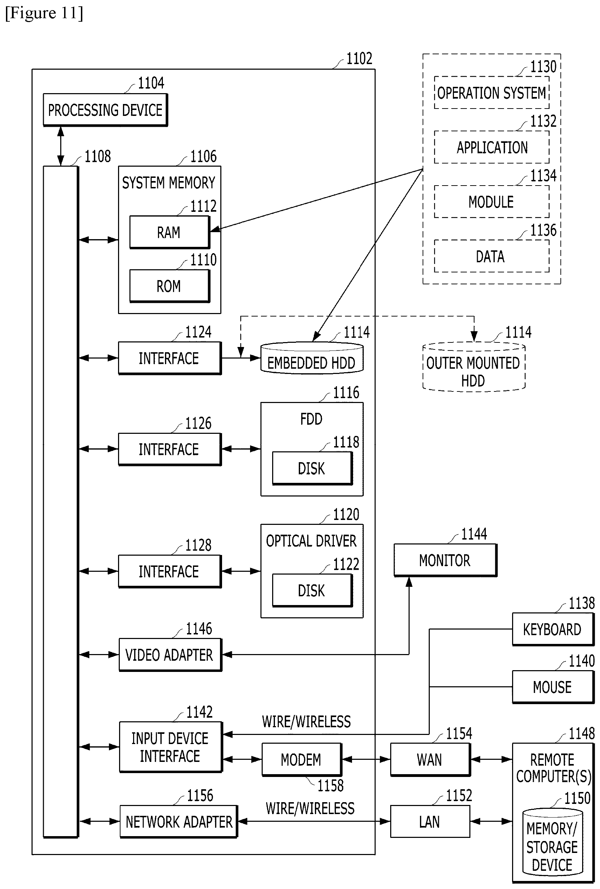

FIG. 11 is a block diagram of a computing device according to some exemplary embodiments of the present disclosure.

FIG. 11 is a simple and general schematic diagram illustrating an example of a computing environment in which the embodiments of the present disclosure are implementable.

The present disclosure has been described as being generally implementable by the computing device, but those skilled in the art will appreciate well that the present disclosure is combined with computer executable commands and/or other program modules executable in one or more computers and/or be implemented by a combination of hardware and software.

In general, a program module includes a routine, a program, a component, a data structure, and the like performing a specific task or implementing a specific abstract data form. Further, those skilled in the art will well appreciate that the method of the present disclosure may be carried out by a personal computer, a hand-held computing device, a microprocessor-based or programmable home appliance (each of which may be connected with one or more relevant devices and be operated), and other computer system configurations, as well as a single-processor or multiprocessor computer system, a mini computer, and a main frame computer.

The embodiments of the present disclosure may be carried out in a distribution computing environment, in which certain tasks are performed by remote processing devices connected through a communication network. In the distribution computing environment, a program module may be located in both a local memory storage device and a remote memory storage device.

The computer generally includes various computer readable media. The computer accessible medium may be any type of computer readable medium, and the computer readable medium includes volatile and non-volatile media, transitory and non-transitory media, and portable and non-portable media. As a non-limited example, the computer readable medium may include a computer readable storage medium and a computer readable transport medium. The computer readable storage medium includes volatile and non-volatile media, transitory and non-transitory media, and portable and non-portable media constructed by a predetermined method or technology, which stores information, such as a computer readable command, a data structure, a program module, or other data. The computer readable storage medium includes a RAM**,** a Read Only Memory (ROM), an Electrically Erasable and Programmable ROM (EEPROM), a flash memory, or other memory technologies, a Compact Disc (CD)-ROM, a Digital Video Disk (DVD), or other optical disk storage devices, a magnetic cassette, a magnetic tape, a magnetic disk storage device, or other magnetic storage device, or other predetermined media, which are accessible by a computer and are used for storing desired information, but is not limited thereto.

The computer readable transport medium generally implements a computer readable command, a data structure, a program module, or other data in a modulated data signal, such as a carrier wave or other transport mechanisms, and includes all of the information transport media. The modulated data signal means a signal, of which one or more of the characteristics are set or changed so as to encode information within the signal. As a non-limited example, the computer readable transport medium includes a wired medium, such as a wired network or a direct-wired connection, and a wireless medium, such as sound, Radio Frequency (RF), infrared rays, and other wireless media. A combination of the predetermined media among the foregoing media is also included in a range of the computer readable transport medium.

An illustrative environment 1100 including a computer 1102 and implementing several aspects of the present disclosure is illustrated, and the computer 1102 includes a processing device 1104, a system memory 1106, and a system bus 1108. The system bus 1108 connects system components including the system memory 1106 (not limited) to the processing device 1104. The processing device 1104 may be a predetermined processor among various commonly used processors. A dual processor and other multi-processor architectures may also be used as the processing device 1104.

The system bus 1108 may be a predetermined one among several types of bus structure, which may be additionally connectable to a local bus using a predetermined one among a memory bus, a peripheral device bus, and various common bus architectures. The system memory 1106 includes a ROM 1110, and a RAM 1112. A basic input/output system (BIOS) is stored in a non-volatile memory 1110, such as a ROM, an EPROM, and an EEPROM, and the BIOS includes a basic routing helping a transport of information among the constituent elements within the computer 1102 at a time, such as starting. The RAM 1112 may also include a high-rate RAM, such as a static RAM, for caching data.

The computer 1102 also includes an embedded hard disk drive (HDD) 1114 (for example, enhanced integrated drive electronics (EIDE) and serial advanced technology attachment (SATA)) - the embedded HDD 1114 being configured for exterior mounted usage within a proper chassis (not illustrated) - a magnetic floppy disk drive (FDD) 1116 (for example, which is for reading data from a portable diskette 1118 or recording data in the portable diskette 1118), and an optical disk drive 1120 (for example, which is for reading a CD-ROM disk 1122, or reading data from other high-capacity optical media, such as a DVD, or recording data in the high-capacity optical media). A hard disk drive 1114, a magnetic disk drive 1116, and an optical disk drive 1120 may be connected to a system bus 1108 by a hard disk drive interface 1124, a magnetic disk drive interface 1126, and an optical drive interface 1128, respectively. An interface 1124 for implementing an outer mounted drive includes, for example, at least one of or both a universal serial bus (USB) and the Institute of Electrical and Electronics Engineers (IEEE) 1394 interface technology.

The drives and the computer readable media associated with the drives provide non-volatile storage of data, data structures, computer executable commands, and the like. In the case of the computer 1102, the drive and the medium correspond to the storage of random data in an appropriate digital form. In the description of the computer readable media, the HDD, the portable magnetic disk, and the portable optical media, such as a CD, or a DVD, are mentioned, but those skilled in the art will well appreciate that other types of computer readable media, such as a zip drive, a magnetic cassette, a flash memory card, and a cartridge, may also be used in the illustrative operation environment, and the predetermined medium may include computer executable commands for performing the methods of the present disclosure.

A plurality of program modules including an operation system 1130, one or more application programs 1132, other program modules 1134, and program data 1136 may be stored in the drive and the RAM 1112. An entirety or a part of the operation system, the application, the module, and/or data may also be cached in the RAM 1112. It will be well appreciated that the present disclosure may be implemented by several commercially usable operation systems or a combination of operation systems.

A user may input a command and information to the computer 1102 through one or more wired/wireless input devices, for example, a keyboard 1138 and a pointing device, such as a mouse 1140. Other input devices (not illustrated) may be a microphone, an IR remote controller, a joystick, a game pad, a stylus pen, a touch screen, and the like. The foregoing and other input devices are frequently connected to the processing device 1104 through an input device interface 1142 connected to the system bus 1108, but may be connected by other interfaces, such as a parallel port, an IEEE 1394 serial port, a game port, a USB port, an IR interface, and other interfaces.

A monitor 1144 or other types of display devices are also connected to the system bus 1108 through an interface, such as a video adaptor 1146. In addition to the monitor 1144, the computer generally includes other peripheral output devices (not illustrated), such as a speaker and a printer.

The computer 1102 may be operated in a networked environment by using a logical connection to one or more remote computers, such as remote computer(s) 1148, through wired and/or wireless communication. The remote computer(s) 1148 may be a work station, a computing device computer, a router, a personal computer, a portable computer, a microprocessor-based entertainment device, a peer device, and other general network nodes, and generally includes some or an entirety of the constituent elements described for the computer 1102, but only a memory storage device 1150 is illustrated for simplicity. The illustrated logical connection includes a wired/wireless connection to a local area network (LAN) 1152 and/or a larger network, for example, a wide area network (WAN) 1154. The LAN and WAN networking environments are general in an office and a company, and make an enterprise-wide computer network, such as an Intranet, easy, and all of the LAN and WAN networking environments may be connected to a worldwide computer network, for example, the Internet.

When the computer 1102 is used in the LAN networking environment, the computer 1102 is connected to the local network 1152 through a wired and/or wireless communication network interface or an adaptor 1156. The adaptor 1156 may make wired or wireless communication to the LAN 1152 easy, and the LAN 1152 also includes a wireless access point installed therein for the communication with the wireless adaptor 1156. When the computer 1102 is used in the WAN networking environment, the computer 1102 may include a modem 1158, is connected to a communication computing device on a WAN 1154, or includes other means setting communication through the WAN 1154 via the Internet. The modem 1158, which may be an embedded or outer-mounted and wired or wireless device, is connected to the system bus 1108 through a serial port interface 1142. In the networked environment, the program modules described for the computer 1102 or some of the program modules may be stored in a remote memory/storage device 1150. The illustrated network connection is illustrative, and those skilled in the art will appreciate well that other means setting a communication link between the computers may be used.

The computer 1102 performs an operation of communicating with a predetermined wireless device or entity, for example, a printer, a scanner, a desktop and/or portable computer, a portable data assistant (PDA), a communication satellite, predetermined equipment or place related to a wirelessly detectable tag, and a telephone, which is disposed by wireless communication and is operated. The operation includes a wireless fidelity (Wi-Fi) and Bluetooth wireless technology at least. Accordingly, the communication may have a pre-defined structure, such as a network in the related art, or may be simply ad hoc communication between at least two devices.

The Wi-Fi enables a connection to the Internet and the like even without a wire. The Wi-Fi is a wireless technology, such as a cellular phone, which enables the device, for example, the computer, to transmit and receive data indoors and outdoors, that is, in any place within a communication range of a base station. A Wi-Fi network uses a wireless technology, which is called IEEE 802.11 (a, b, g, etc.) for providing a safe, reliable, and high-rate wireless connection. The Wi-Fi may be used for connecting the computer to the computer, the Internet, and the wired network (IEEE 802.3 or Ethernet is used). The Wi-Fi network may be operated at, for example, a data rate of 11 Mbps (802.11a) or 54 Mbps (802.11b) in an unauthorized 2.4 and 5 GHz wireless band, or may be operated in a product including both bands (dual bands).

Those skilled in the art may appreciate that information and signals may be expressed by using predetermined various different technologies and techniques. For example, data, indications, commands, information, signals, bits, symbols, and chips referable in the foregoing description may be expressed with voltages, currents, electromagnetic waves, magnetic fields or particles, optical fields or particles, or a predetermined combination thereof.

Those skilled in the art will appreciate that the various illustrative logical blocks, modules, processors, means, circuits, and algorithm operations described in relationship to the embodiments disclosed herein may be implemented by electronic hardware (for convenience, called "software" herein), various forms of program or design code, or a combination thereof. In order to clearly describe compatibility of the hardware and the software, various illustrative components, blocks, modules, circuits, and operations are generally illustrated above in relation to the functions of the hardware and the software. Whether the function is implemented as hardware or software depends on design limits given to a specific application or an entire system. Those skilled in the art may perform the function described by various schemes for each specific application, but it shall not be construed that the determinations of the performance depart from the scope of the present disclosure.

Various embodiments presented herein may be implemented by a method, a device, or a manufactured article using a standard programming and/or engineering technology. A term "manufactured article" includes a computer program, a carrier, or a medium accessible from a predetermined computer-readable storage device. For example, the computer-readable storage medium includes a magnetic storage device (for example, a hard disk, a floppy disk, and a magnetic strip), an optical disk (for example, a CD and a DVD), a smart card, and a flash memory device (for example, an EEPROM, a card, a stick, and a key drive), but is not limited thereto. Further, various storage media presented herein include one or more devices and/or other machine-readable media for storing information.

It shall be understood that a specific order or a hierarchical structure of the operations included in the presented processes is an example of illustrative accesses. It shall be understood that a specific order or a hierarchical structure of the operations included in the processes may be rearranged within the scope of the present disclosure based on design priorities. The accompanying method claims provide various operations of elements in a sample order, but it does not mean that the claims are limited to the presented specific order or hierarchical structure.

The description of the presented embodiments is provided so as for those skilled in the art to use or carry out the present disclosure. Various modifications of the embodiments may be apparent to those skilled in the art, and general principles defined herein may be applied to other embodiments without departing from the scope of the present disclosure. Accordingly, the present disclosure is not limited to the embodiments suggested herein, and shall be interpreted within the broadest meaning range consistent to the principles and new characteristics presented herein.

## Claims

1. A method for providing menstrual related information, which is performed by a computing device, the method comprising:
obtaining biometric information including a plurality of predetermined electrocardiogram variable information, the plurality of electrocardiogram variable information including time series data;
obtaining user question and answer information corresponding to the plurality of electrocardiogram variable information; and
generating, by an analysis model, menstrual cycle prediction information based on the biometric information and the user question and answer information.

2. The method of claim 1, wherein the plurality of electrocardiogram variable information includes at least one of RR Intervals (RRI) information, heart rate (HR) information, respiration rate (RR) information, ST level information, standard deviation of all NN intervals (SDNN) information, square root of the mean of the sum of the squares of differences between adjacent NN intervals (RMSSD) information, number of NN intervals differing by more than 50 ms (NN50) information, ratio of NN50 (pNN50) information, and standard deviation of differences between neighboring NN intervals (SDSD) information.

3. The method of claim 1 or 2, wherein the user question and answer information includes at least one of sleep disorder question and answer information, depression question and answer information, anxiety question and answer information, and stress question and answer information.

4. The method of claim 2, wherein the analysis model includes an ensemble learning model using a plurality of prediction variable combinations including at least some of the plurality of electrocardiogram variable information and the user question and answer information.

5. The method of claim 1 or 4, wherein the analysis model is trained to generate information predicting a transition from a follicular phase to a luteal phase when identifying a decreasing trend of the RRI information based on the RRI information.

6. The method of claim 1 or 4, wherein the analysis model is trained to generate information predicting a transition from a menstrual phase to the follicular phase when identifying a retention decreasing trend of the HR information,
is trained to generate information predicting a transition from the follicular phase to a fertile phase when identifying an increasing trend of HR information, and
is trained to generate information predicting a transition to the luteal phase when identifying a highest level trend of the HR information.

7. The method of claim 1 or 4, wherein the analysis model is trained to generate information predicting a transition from the menstrual phase to the follicular phase when identifying a decreasing trend of the RR information,
is trained to generate information predicting a transition to the fertile phase when identifying an additional decreasing trend of the RR information, and
is trained to generate information predicting a transition from the luteal phase when identifying an increasing trend of the RR information.

8. The method of claim 1 or 4, wherein the analysis model is trained to generate information predicting a transition to the follicular phase when identifying a highest level trend of the ST level information, and
is trained to generate information predicting a transition to the luteal phase when identifying a lowest level trend of the ST level information.

9. The method of claim 1 or 4, wherein the analysis model is trained to generate information predicting a transition from the menstrual phase to the follicular phase when identifying a retention trend of the SDNN information,
is trained to generate information predicting a transition from the follicular phase to the fertile phase when identifying a decreasing trend of the SDNN information, and
is trained to generate information predicting a transition to the luteal phase when identifying a maximum decreasing trend of the SDNN information.

10. The method of claim 1 or 4, wherein the analysis model is trained to generate information predicting a transition to the luteal phase when identifying a maximum decreasing trend of the RMSSD information.

11. The method of claim 1 or 4, wherein the analysis model is trained to generate information predicting a transition from the follicular phase to the luteal phase when identifying a decreasing trend of the NN50 information.

12. The method of claim 1 or 4, wherein the analysis model is trained to generate information predicting a transition from the follicular phase to the luteal phase when identifying a decreasing trend of the pNN50 information.

13. The method of claim 1 or 4, wherein the analysis model is trained to generate information predicting a transition from the menstrual phase to the follicular phase when identifying a retention trend of the SDSD information.

14. The method of claim 1 or 4, wherein the user question and answer information includes sleep disorder question and answer information, and
the analysis model is trained to generate information predicting a transition to the luteal phase when identifying an increasing trend of the sleep disorder question and answer information.

15. The method of claim 1 or 4, wherein the user question and answer information includes depression question and answer information, and
the analysis model is trained to generate information predicting a transition to the luteal phase when identifying an increasing trend of the depression question and answer information.

16. The method of claim 1 or 4, wherein the user question and answer information includes anxiety question and answer information, and
the analysis model is trained to generate information predicting a transition to the luteal phase when identifying an increasing trend of the anxiety question and answer information.

17. The method of claim 1 or 4, wherein the user question and answer information includes stress question and answer information, and
the analysis model is trained to generate information predicting an ovulation delay when identifying an increasing trend of the stress question and answer information.

18. The method of claim 1 or 4, wherein the obtaining of the user question and answer information corresponding to the plurality of electrocardiogram variable information includes
providing a user interface, capable of inputting the user question and answer information, corresponding to time points of obtaining the plurality of electrocardiogram variable information.

19. The method of claim 18, wherein the obtaining of the user question and answer information corresponding to the plurality of electrocardiogram variable information includes
providing a user interface indicating whether to input the user question and answer information corresponding to the time points of obtaining the plurality of electrocardiogram variable information.

20. The method of claim 1, wherein the plurality of electrocardiogram variable information includes RRI information, HR information, RR information, ST level information, SDNN information, RMSSD information, NN50 information, pNN50 information, and SDSD information.

21. The method of claim 20, wherein the user question and answer information includes sleep disorder question and answer information, depression question and answer information, anxiety question and answer information, and stress question and answer information.

22. The method of claim 4, wherein the analysis model uses a random forest algorithm.

23. A computer program stored in a computer readable medium, wherein the computer program includes instructions allowing one or processors to perform a method for providing menstrual related information, the method comprising:
obtaining biometric information including a plurality of predetermined electrocardiogram variable information, the plurality of electrocardiogram variable information including time series data;
obtaining user question and answer information corresponding to the plurality of electrocardiogram variable information; and
generating, by an analysis model, menstrual cycle prediction information based on the biometric information and the plurality of user question and answer information.

24. A computing device performing a method for providing menstrual related information, the computing device comprising:
a memory including computer-executable components; and
a processor executing following computer-executable components stored in the memory,
wherein the processor is configured to
obtain biometric information including a plurality of predetermined electrocardiogram variable information, the plurality of electrocardiogram variable information including time series data,
obtain user question and answer information corresponding to the plurality of electrocardiogram variable information, and
generate, by an analysis model, menstrual cycle prediction information based on the biometric information and the plurality of user question and answer information.
